(19) Europäisches Patentamt
European Patent Office
Office européen des brevets

(11) **EP 1 435 920 B1**

(12) **EUROPEAN PATENT SPECIFICATION**

(45) Date of publication and mention
of the grant of the patent:
**09.12.2009 Bulletin 2009/50**

(51) Int Cl.:
***A61K 9/22*** *(2006.01)*

(21) Application number: **02802414.9**

(22) Date of filing: **30.08.2002**

(86) International application number:
**PCT/US2002/027745**

(87) International publication number:
**WO 2003/037305 (08.05.2003 Gazette 2003/19)**

(54) **Implantable drug delivery device with flow regulator**

Implantierbare Vorrichtung zur Arzneimittelverabreichung mit Flussregulator

Dispositif implantable pour l'administration de médicaments avec regulateur de debit

(84) Designated Contracting States:
**AT BE BG CH CY CZ DE DK EE ES FI FR GB GR IE IT LI LU MC NL PT SE SK TR**

(30) Priority: **19.09.2001 US 323630 P**

(43) Date of publication of application:
**14.07.2004 Bulletin 2004/29**

(73) Proprietor: **Durect Corporation**
**Cupertino, CA 95014-4166 (US)**

(72) Inventor: **THEEUWES, Felix**
**Los Altos Hills, CA 94022 (US)**

(74) Representative: **Price, Nigel John King et al**
**J.A. Kemp & Co.**
**14 South Square**
**Gray's Inn**
**London WC1R 5JJ (GB)**

(56) References cited:
| | |
|---|---|
| EP-A2- 0 564 321 | WO-A-00/66204 |
| WO-A-01/26708 | WO-A-86/02846 |
| WO-A2-01/56633 | DE-A1- 19 812 436 |
| US-A- 3 659 600 | US-A- 4 220 153 |
| US-A- 5 011 472 | US-A- 5 062 841 |
| US-A- 5 368 571 | US-A- 6 152 181 |
| US-A1- 2001 044 620 | |

**Description**

The present invention relates to implantable drug delivery devices that control of the release of active agents therefrom.

Few therapeutic regimens involve administration of a single dose of a selected drug. Instead, most therapies require administration of multiple doses. Where the therapy requires parenteral delivery of the drug, the patient can be subjected to the substantial discomfort and inconvenience of repeated injections. This can be particularly problematic where the condition or disease to be treated requires long-term therapy. The repeated injections required for such long-term therapy not only meet with difficulties associated with patient compliance, but also can lead to collapse of veins and substantial tissue damage. Parenteral drug delivery typically also requires administration of a bolus of drug in order to provide for an effective drug concentration at the desired treatment site and/or to provide for an adequate systemic levels for an acceptable period of time (*e.g.*, as in treatment of diabetes with insulin). Delivery of a drug bolus not only requires delivery of a greater amount of drug, thus driving up the cost of therapy, but can also be associated with undesirable side effects.

Many diseases or indications require long term, chronic delivery of drugs or agents to a patient, e.g., cancer, arthritis, heart disease, etc. Long term delivery of drugs or agents can be accomplished by use of drug delivery systems comprising drug delivery devices which may be implanted in a patient's body or retained externally. Drug delivery systems can also deliver drugs or agents to a targeted site within the body via catheters attached to drug delivery devices with the distal end of such catheters placed at the desired site of delivery in the body, with the catheter acting a conduit for the drug or desired agent from the drug delivery device to the desired site of delivery in the body. Drug delivery devices which have adjustable drug delivery rates are known in the art (see, *e.g.*, U.S. Pat. No. 4,692,147). However, such devices with variable or programmable drug delivery rates often include complex mechanical elements which may make such drug delivery devices bulky and subject to failure. Constant drug delivery devices provide for delivery of drug at a preselected, substantially non-fluctuating rate, thus providing for predictability of the dose delivered. However, constant drug delivery devices have the limitation that the rate of drug or agent delivered cannot be readily adjusted, particularly where the drug delivery device is implanted in the body. The ability to readily alter the rate at which drug is administered is often desirable in that it provides flexibility in a therapeutic regimen, and in certain cases, may be a requirement in certain therapies. For example, the drug requirements of a patient may not be ascertainable prior to the commencement of a therapy (*e.g.*, dose titration may be required to determine appropriate dosage), or a patient may require increasing doses (*e.g.*, due to development of tolerance) or decreasing doses (*e.g.*, as the patient gets well). In a constant drug delivery device, adjusting the rate of drug delivery can require the removal of the device from the body of a patient and/or detachment from a drug delivery system (*e.g.*, detachment from a catheter) and adjustment or even replacement of the device.

There is thus a need in the field for a mechanism which allows for adjustment of the rate of a drug delivery device yet obviates the need for complex or bulky regulatory elements associated with the drug delivery device. The present invention addresses this problem.

WO 01/26708 discloses the pre-characterising portion of claim 1.

The invention is a membrane controlled delivery device having at least one membrane adapted to facilitate membrane controlled delivery, and having a regulator to change a transport area of a combination of the regulator and at least one membrane.

The membrane controlled delivery device may be implanted and adjusting of the regulator may still be performed after the implantation by remotely adjusting the regulator from a location external of a site of the implantation.

The adjusting may comprise changing an available area, of the at least one membrane, through which diffusion electrodiffusion, electroosmosis or osmosis occurs, such that more or less of the area is available for the transport of fluid.

First and second cover members may be included in the regulator, where the first cover member has a flow resistance much greater than a flow resistance of at least one membrane, and the second cover member has a flow resistance much less than the flow resistance of at least one membrane. In this case, the adjustment includes moving the first and second cover members relative to vary a portion of the area of the at least one membrane covered by the first cover member relative to a portion of the area covered by the second cover member.

Remote adjustment may be performed magnetically, or by telemetry.

In another example, pressure is applied to drive fluids to adjust the cover by a first cover member relative to a second cover member. Other drivers are provided for changing the coverage ratio, such as a screw threaded cap, for example.

The cover members may be fluid. At least one of the cover members may be a magnetic fluid. A magnetic driver may be provided between first and second cover members.

An implantable drug delivery device is disclosed to include a body adapted to contain at least one drug therein for dispensing therefrom; at least one membrane adapted to selectively pass one or more agents therethrough for driving delivery of the drug from the device; and at least one cover member movable with respect to at least one membrane to cover at least a portion of at least one membrane to vary the cross sectional area exposed for transport of the one or more agents.

The cover member may include a cover member with substantially infinite resistance to transport of the one or more agents, wherein movement of the cover member over the at least one membrane effectively reduces the cross-sectional area effective for fluid transport therethrough.

Additionally, a second cover member having substantially insignificant resistance to transport of the one or more agents, relative to a resistance of the at least one membrane may be provided adjacent the first cover member of substantially infinite resistance. In this way, the second cover member covers any portion of the cross-sectional area not covered by the first cover member.

The cover member may contain any grade of resistance from zero to 100 percent. At least one membrane may include a pair of membranes aligned in parallel and defining a gap therebetween, where the first and second covers may pass through the gap to effectively cover the corresponding cross-sectional areas of each of the membranes.

Each cover member may be a fluid. A pair of fluid cover members may be provided wherein at least one of the fluids is a magnetic fluid. First and second fluid cover members are generally immiscible with one another.

The regulator may be remotely adjusted from a location external of a site of the implantation.

The adjusting may comprise changing an available area, of the at least one membrane, through which diffusion, osmosis, electroosmosis, pressure filtration (PF), electro diffusion,or combinations thereof occur.

The regulator may comprise a first cover and a second cover. The first cover having a flow resistance much greater than a flow resistance of the at least one membrane and the second cover may have a flow resistance much less than the flow resistance of the at least one membrane. The adjusting in this example may comprise moving the first and second covers relative to vary a portion of the area of the at least one membrane that is covered by the first cover relative to a portion of the area covered by the second cover.

The adjusting of such first and second covers may be accomplished magnetically or telemetrically.

The first and second covers may be fluid covers, and adjustment thereof may comprise applying fluid pressure to drive the first and second covers.

The remote adjusting may be magnetically driven and/or telemetrically controlled from a location remote from the device.

An implantable drug delivery device is described to include a body adapted to contain at least one drug therein for dispensing therefrom: at least one membrane adapted to selectively pass one or more liquids therethrough, the at least one membrane having a cross-sectional area adapted to be exposed to the one or more liquids for transport thereof, and having predefined transport characteristics with regard to transport of the one or more liquids therethrough, and at least one cover member movable with respect to the at least one membrane to cover at least a portion of the at least one membrane to vary the cross sectional areas exposed for transport of the one or more liquids.

The at least one cover member may include a cover member with substantially infinite resistance to transport of the one or more liquids, and in this case movement of the cover member over the membrane effectively reduces the cross-sectional area.

In addition to a cover member with substantially infinite resistance used as a first cover member, the device may further include a second cover member having substantially insignificant resistance to transport of the one or more liquids, relative to a resistance of the at least one membrane. The second cover member may be located adjacent the first cover member and covers any portion of the cross-sectional area not covered by the first cover member.

The at least one membrane allows inflow of fluids to drive the at least one drug from the body.

The at least one membrane may comprise a pair of membranes aligned in parallel and defining a gap therebetween, the first and second covers being adapted to pass through the gap to effectively cover the cross-sectional area.

The device may further define a labyrinth between the pair of membranes, which is adapted to channel the covers therethrough.

The pair of membranes may have equal cross-sectional areas, each of which are effected simultaneously by the cover members.

The at least one cover member may comprise a fluid. The at least one cover member may comprise a magnetic fluid. Using first and second cover members, the first cover member may comprise a first fluid and the second cover member may comprise a second fluid different from the first fluid. At least one of the first and second fluids may be a magnetic fluid.

The at least one cover member may be driven remotely, after implantation of the device in an implantation site, from a location external of the implantation site.

The at least one cover member may be driven magnetically and/or telemetrically.

These and other objects, advantages, and features of the invention will become apparent to those persons skilled in the art upon reading the details of the invention as more fully described below.

The invention will now be further described, by way of example only, with reference to the accompanying drawings, in which:-

Fig. 1 is a schematic illustration of a flow regulator according to the present invention.

Fig. 2 is a partial sectional view of an implantable drug delivery device equipped for flow regulation according to the present invention.

Fig. 3 is a top view of a device showing a labyrinthine configuration for adjusting the flow therethrough according to the present invention.

Fig. 4 is a partial sectional view of another example of an implantable drug delivery device equipped for flow regulation according to the present invention.

Fig 5 is a partial section view of a still further example of an implantable drug delivery device equipped for flow regulation according to the present invention.

Fig. 6 is a sectional, partial view of an implantable drug delivery device emplying expandable reservoirs used to drive the positioning of cover members.

Fig. 7 is a sectional, partial view of another example of an implantable drug delivery device equipped for flow regulation according to the present invention.

Before the present devices are described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

**Definitions**

"Drug delivery device" is meant to refer to any device or combination of devices that can provide for transfer of drug from a drug reservoir, through one or more membranes and to a treatment site. "Drug delivery device" thus encompasses, for example, a drug delivery device (e.g., implantable pump) with a flow regulator of the invention; a drug delivery device, flow regulator, and drug delivery catheter combination; and the like.

The terms "drug," "therapeutic agent," or "active agent" as used herein are meant to encompass any substance suitable for delivery to a treatment site of a subject, which substances can include pharmaceutically active drugs, as well as biocompatible substances that do not exhibit a pharmaceutical activity in and of themselves, but that provide for a desired effect at a treatment site, e.g., to flush or irrigate a treatment site (e.g., saline), provide for expression or production of a desired gene product (e.g., pro-drug, polynucleotide, and the like), etc. In general, "drug" and the like are used to encompass any drug administered by parenteral administration, particularly by injection (e.g., intravascularly, intramuscularly, subcutaneously, intrathecally, etc, or by insertion in any body cavity or orifice). Drugs compatible for delivery using the devices and methods of the invention are discussed below, and are readily apparent to the ordinarily skilled artisan upon reading the disclosure provided herein. Drugs may optionally be provided in combination with pharmaceutically acceptable carriers and/or other additional compositions such as antioxidants, stabilizing agents, permeation enhancers, etc.

The term "agent" includes any drug, electrolyte, water or liquid that may be used with the present invention, either to be dispensed from a device according to the present invention, or in use for driving another agent from the device as it enters the device through a membrane.

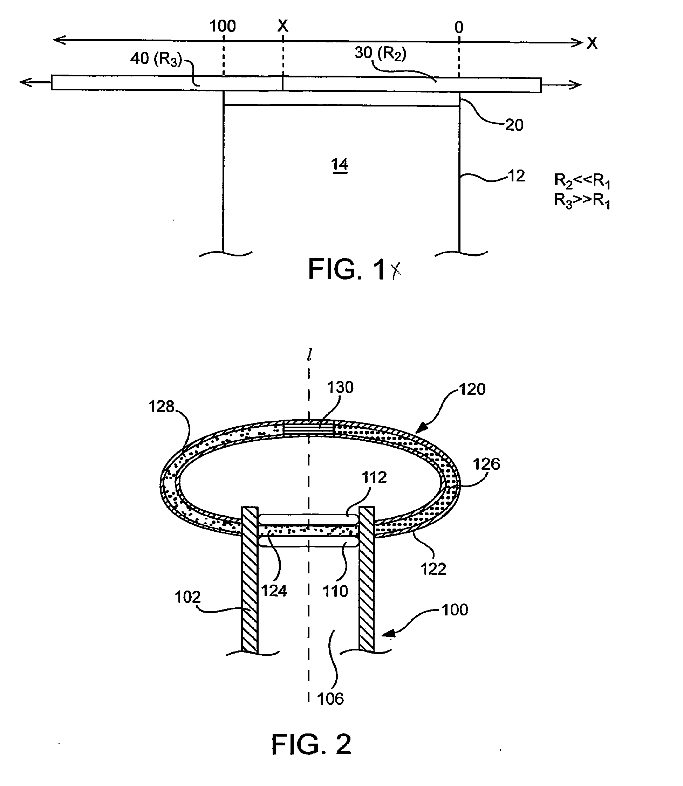

The present invention encompasses devices for regulating the rate of drug delivery from a drug delivery device through at least one membrane. As illustrated in the schematic of Fig. 1, the invention pertains to regulation of drug delivery rate from a drug delivery device having a reservoir 12 containing one or more active agents or drugs 14 that are driven through membrane 20 for delivery to a treatment site. The driving force for driving drug 14 through membrane 20 may be osmosis, diffusion, electrodiffusion, electroosmosis, electrochemical for example, although the present invention is not limited only to implantable devices driven by these modalities, but may also include actively driven devices (e.g., motor-driven) for example, or any other membrane modulated device which delivers drug to a treatment site.

The rate of transport of an agent 14 through membrane 20 is governed by the thickness, area and transport characteristics (e.g., permeability coefficient, solubility coefficient, diffusion coefficient, depending upon the type of transport occurring) of the membrane 20 with respect to the types of molecules being transported. The present invention controls the rate of transport through membrane 20 by varying the effective area of the membrane that is available for transport, while holding the thickness and transport characteristics constant.

Referring again to Fig. 1, if we assume the area of membrane 20 that is available for the agent 14 to pass through is $A_1$ and that the resistance of membrane 20 to mass transport is $R_1$, then a flow rate of agent 14 through membrane 20 is a function of the area $A_1$ as well as the resistance $R_1$ to the flow of the agent (which, as noted above, is further a function of the thickness of the membrane 20 and transport characteristics of the material making up the membrane 20 with respect to the agent (s) to be transported and the method of transportation.

A serial arrangement of membranes, surfaces, or covers 40 and 30 are provided to contact the external surface of the membrane 20 where all of the membranes have the same width. In this example, membrane or cover 30 has a

resistance $R_2$ which is very much less than $R_1$, so much less as to be effectively negligible with respect to restricting the transport of agent 14 therethrough, relative to the restrictive effect by membrane 20. Membrane or cover 40, on the other hand, has a resistance $R_3$ which is very much greater than $R_1$, so much more as to effectively block the transport of agent 14 therethrough, relative to the rate of flow allowed by membrane 20.

As noted above, covers 30 and 40 are serially connected. Further, covers 30 and 40 are configured for translation over the membrane 20, in the directions indicated by the left and right pointing arrows in Fig. 1. Although it is more convenient to arrange the covers 30 and 40 against the outside surface of membrane 20, the same type of control could be achieved, albeit more painstakingly (i.e., requiring a more complicated mechanical arrangement) by arranging the covers 30 and 40 against the inside surface of the membrane 20. Upon translation all the way to the left in Fig. 1, membrane 20 will be completely covered by membrane 30 only. In this configuration, since the resistance $R_2$ of membrane 30 is very much less than $R_1$ and has a negligible effect on the mass. transport, the total resistance will remain at a value of substantially $R_1$, i.e., $R_1 + R_2 \approx R_1$. Upon translation of the members 30 and 40 all the way to the right in Fig. 1 on the other hand, membrane 20 will be completely covered by membrane 40 only. In this configuration, since the resistance $R_3$ of membrane 40 is very much greater than $R_1$ so as to substantially prohibit any transport of agent 14 therethrough, the total resistance will be $R_3$, which, for purposes of discussion is effectively $\infty$ i.e., $R_1 + R_3 \approx R_3 \approx \infty$.

At positions between the two extreme positions of the members 30 and 40, membrane 20 will be covered by varying proportionalities of the two members 30 and 40. By varying the effective area of membrane 20 covered by member 30 relative to the area of membrane 20 covered by member 40, one can effectively control the total resistance to mass transport between the values of $R_1$ and $\infty$. The effective delivery or transport rate of agent 14 can thus be effectively controlled to desired values between the maximum flow rate allowed (i.e., at resistance $R_1$) and zero (i.e., at resistance of $\infty$).

For example, the resistance of a diffusional system can be controlled by controlling the fraction of the total area $A_1$ which is covered by member 30 according to the following formula:

$$(\mathbf{dm/dt})_x = \mathbf{X} \cdot \mathbf{A_1} \cdot \mathbf{D} \cdot \mathbf{K} \ \mathbf{c./h})$$

where $(dm/dt)_x$ is the mass transport rate when members 30 and 40 are positioned at a given position "x"; (along the x axis as depicted in Fig. 1);

X is a multiplier (running from 0 to 100 from right to left as shown in Fig. 1) indicating the portion of the area $A_1$ that is covered by member 30. For example, if member 30 covers 75% of the area of membrane 20, then b would be equal to .75;

D is the diffusion coefficient of drug in membrane 20 and is independent of X;

K is the partition coefficient of drug between membrane 20 and membrane 14, K also being independent of X;

C is the concentration of drug in medium 14, which is also independent of X; and

h is the membrane thickness of membrane 20.

The resistance of any delivery system with constant driving force be it diffusional, electrochemical, electroosmotic , osmotic, or the like, can be described by:

$$(\mathbf{dm/dt})_x = \mathbf{X} \ (\mathbf{dm/dt})_{\mathbf{max}}$$

where $(dm/dt)_x$ is the mass transport rate when members 30 and 40 are positioned at a given position "x"; on the x axis (see Fig. 1);

X is a multiplier indicating the fraction of the area $A_1$ that is covered by member 30. For example, if member 30 covers 75% of the area of membrane 20, then X would be equal to .75; and

$(dm/dt)_{max}$ is the delivery rate when X is at the 100% mark, or fully engaged at position "x".

It is noted that the present invention should not be limited to arrangements where member 20, members 30 and 40 are limited to effective resistances of substantially zero and substantially infinity, respectively. Those are only the most straightforward examples where the mass transfer rate is varied by varying only the effective area through which the transport is accomplished. The same type of control can be practiced using members 30' and 40', where the resistance $R_{30}$ is non zero and not infinity and the resistance $R_{40}$ of member 40' is also not zero or infinity. The resistance of membrane 20 is $R_{20}$. In this type of arrangement, the effective resistance to mass transport can be defined by the total resistance of the membrane composite, designated as Rt, where:

$$Rt = R_{20} + X\,R_{30} + (100\text{-}x)\,R_{40.}$$

Thus, the rate of drug delivery can be controlled even after implantation of a drug delivery device, by externally and remotely controlling the rate of transport through one or more membranes, which directly effects the rate of drug delivery. For example, membrane area regulation can be effected for intermittent delivery of drugs (e.g. for ten minutes out of every hour) or continuous rates of delivery can be changed at will.

Fig. 2 is an example of an implantable drug delivery device 100 equipped for control of drug release therefrom by variation of the area accessible for conducting delivery through membranes 110 and 112. Device 100 includes a substantially fluid impermeable body 102 which may be made of titanium, for example, or other equivalent bicompatible, substantially fluid impermeable, structural material known in the art, including polymeric materials such as polyethylene, silicone rubbers, and other known equivalent and biocompatible metals, plastics and composites known in the art Defined within the body 102 is a reservoir 106 from which agent is exchanged through membranes 110 and 112. The agent can be a drug or driving fluid such as water and/or electrolyte. Once placed in an environment of use, such as within a body cavity or within body tissue, agent transport is initiated between the environment and the chamber 106 through membranes 110,112. Membranes used in diffusion systems will allow transfer of drug from the reservoir and may be made of, for example, ethylene vinylacetates, polyurethanes, silastic, microporous cellulose acetate, etc. For electro chemical systems, the membranes may be NAFION™ (available from E.I. DuPont Nemours Co., Wilmington, Delaware) or RAIPORE™ (e.g., RAIPORE™ 5010, manufactured by RAI Research Corp., Hauppauge, N.Y.) Other acceptable ion exchange membranes or other ion exchange membranes known and used for this purpose are identified in U.S. Patent No. 5,169,383. Further information regarding electrochemical systems is disclosed in U.S. Patent Nos. 5,951,538; 5,567,287; 4,886,514; 5,593,552; 5,538,605; 5,454,922; 5,707,499; and 5,855,761.

For use in osmotic systems, the membranes may comprise cellulose acetate, polyurethanes, polyethylene, ethylene vinyl, natural rubber, polyamides, polymethylmethacrylate, polyvinylidene chloride, polyvinyl chloride, styrene acrylonitrile, styrene butadiene, polystyrene, block copolymer, polyolefin latex rubber fluoroelastomers, puma, acrylonitrile, polysulfone, alophinics, polyesters (e.g., SARAN resin available from Dow Corning), or acrylics, or blends thereof for example). Other acceptable materials for use in osmotic systems are identified in U.S. Patent No. 4,865,845.

A membrane area regulator 120 is provided for controlling the rate of transport through the membranes 110 and 112. In this example, tubing 122 is provided to seal with a predefined area 124 between membranes 110 and 112 and is configured so as to be capable of circulating two different cover substances 126 and 128 between the membranes 110,112 to partially or totally cover the area through which osmosis occurs. In Fig. 2, cover fluid 128 is shown as completely filling the area 124 between membranes 110 and 112. If, for example, the resistance of fluid 128 is negligible compared to the resistance provided by membranes 110 and 112, then the configuration in Fig. 2 shows the configuration which will achieve a maximum rate of transport (and thus, a maximum rate of drug delivery) for the device 100.

The fluid 126 may be of substantially greater resistance than the combined resistance of membranes 110 and 112, and may be so great as to substantially prevent delivery therethrough. In such a case, positioning of the fluid 126 to completely fill the area 124 between membranes 110 and 112 would effectively reduce the rate of transport (and drug delivery) to zero. The effective delivery rate of drug from the device 100 can thus be effectively controlled to desired values between the maximum flow rate allowed (i.e., at the setting shown in Fig. 2) and zero. Fluids 126 and 128 are selected to be substantially immiscible. Examples of fluids that may function as a low resistance fluid 128 are water, saline and the like. High resistance fluids 126 may include oils, such as vegetable oils and other biocompatible oils (preferred, or other oils which are essentially immiscible in water such as silicon oil and hydrocarbons oils, or mercury, etc.

Relative positioning of the cover fluids may be performed by driving adjustment piston 130 to a desired position. Adjustment piston 130 forms a fluid tight seal with tubing 120(which may be formed of plastic, such as PVC, polyurethane, air, gas, polypropylene, polyethylene, EVA, or other known biocompatible polymers, for example), such that when adjustment piston 130 is translated with respect to the tubing 122, it drives one or the other of fluids 126,128 (depending upon the direction of translation), which in turn drives the second fluid to follow the adjustment piston, thereby completing the circuit and repositioning the respective positions of the fluids 126,128 within the tubing 122.

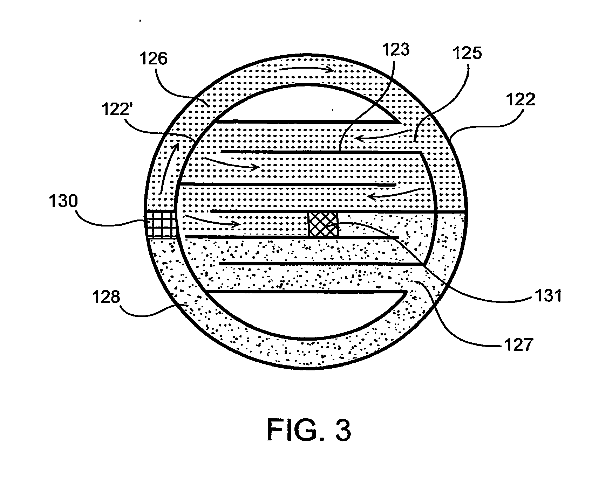

Figure 3 is a top view of a device for regulating flow according to the present invention. The device has an inner circle 122' divided in labyrinth form within outer circle 122 The space between 122' and 122 is filled with fluids 126 and 128 which are separated by partitions 130 and 131. Partition 130 may be a magnetic piston, for example. Partition 131 may be a meniscus or gas or other divider dividing immiscible liquids 126 and 128. Movement of piston 130 relatively upward in Fig. 3 will drive a relatively greater portion of fluid 126 into the labyrinth 123 through passage 125. At the same time, an equal portion of fluid 128 will be driven from the labyrinth 123 through passage 127 by the action of the movement of partition 131 due to the fact that both liquids 126 and 128 are relatively incompressible. By selecting fluid 126 to be relatively impervious to the agent to be transported, the afore-described movement of the piston 130 will increase the resistance through the circular face 122' to transport of the agent therethrough. The circle 122' is covered on both sides of the labyrinth with a membrane material (not shown) that is permeable to the agent to be transported, and the fluids 126,128 are contained and channeled between the membrane materials and in the labyrinth 123.

In the example shown, the adjustment piston 130 is a permanent magnet, although the present invention is not to be limited to such. By configuring adjustment piston 130 with magnetic properties, control of the relative positioning of the fluids 126 and 128 can be effected remotely, e.g., outside the skin of a patient into which the device 100 has been subcutaneously implanted. Or is such control limited to subcutaneous implantation, but would also apply to intramuscular implantation, intracavity placement, and other implantation sites. In practice, an operator would simply apply a magnet near or against the skin above the location of the adjustment piston, and then move the magnet to the desired location of repositioning to set the desired area of cover of liquid 128 relative to the area of cover of liquid 126, both with respect to the membranes 110 and 112, thereby setting the rate of drug delivery to a desired value between and including zero and the maximum rate obtainable by the device 100.

This type of control is very useful in situations where a constant flow of drug to the treatment site is not necessary or desirable. In such situations, the flow of drug can be shut down for desired times, even after implantation, by simply applying a magnet, as indicated above, and moving it to the rightmost position to completely block the area 124 with fluid 126. Control can also be implemented to increase or decrease the release of drug, at selected rates within the range indicated above.

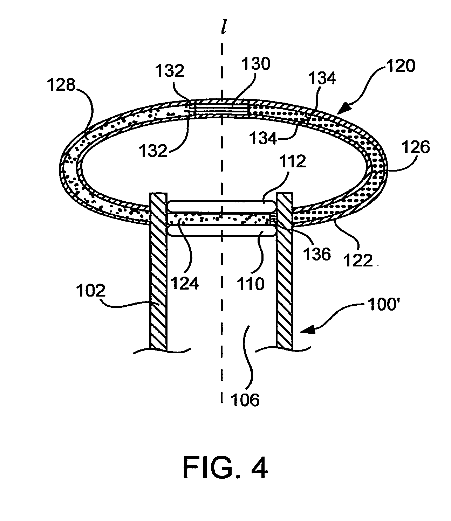

Fig. 4 is another example of an implantable, osmotically driven drug delivery device 100' which is very similar to the example shown in Fig. 2, with the addition of some optional features that aid in the control of membrane area regulation. These optional features are not limited to use with the example shown in Fig. 4, but may be employed with any arrangement employing liquid covers. In this example, stops 132 and 134 are provided within the tubing 122 to physically prevent the adjustment piston 130 from traveling beyond positions that correspond to maximum drug rate delivery (stops 132) and zero drug rate delivery (stops 134). Each of stops 132 and 134 may be formed as a pair of protrusions extending inwardly from the inner wall of the tubing 122, an annular projection extending inwardly from the inner wall of the tubing 122, or other obstruction that would impede the travel of adjustment piston 130 therepast.

Although fluids 126 and 128 are substantially immiscible with one another, a dividing piston may be provided at the interface between the fluids 126 and 128. Dividing piston 136 is configured to seal with the inner wall of tubing 122, but to also translate with the driving of fluids 126 and 128 by adjustment piston 130, so as to substantially prevent any mixing of the fluids or shearing of the fluids one underneath the other, during repositioning of the fluids by the adjustment piston. Dividing piston 136, like fluid 126, has a substantially infinite resistance to diffusion and therefor effects flow rate in a substantially identical manner to that of fluid 126.

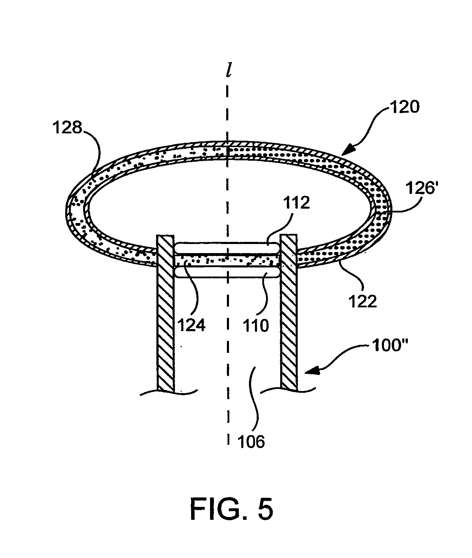

Fig. 5 is another example of an implantable, osmotically driven drug delivery device 100" which is very similar to the examples shown in Figs. 2 and 4. However, in this example, fluid 126' itself is magnetic, and can therefor be remotely positioned using a magnet external to the body of the organism into which the device has been implanted. Fluid 126', like the previous embodiments is substantially immiscible with fluid 128 and has a substantially infinite resistance to diffusion, thereby effectively blocking diffusion across any portion of the areas of membranes 110 and 112 which are obstructed by fluid 126' as it is positioned in space 124. Examples of such a magnetic fluid 126' include commercially available magnetic fluids which generally comprise a carrier liquid and small magnetic particles held in suspension by a surface active layer, a surfactant. Such carrier liquids may include hydrocarbon oils and PFPEs, for example. The most common source of magnetic particles is iron oxide, ferrite, because it oxidizes less readily than other metal oxides. These magnetic particles are stabilized within the carrier fluid so that they are maintained in suspension under all conditions. Although not needed for driving in this example, a piston (not shown) may be placed at either or both of the junctions between fluids 126' and 128 to ensure that shearing of one fluid under the other during repositioning of the fluids does not occur.

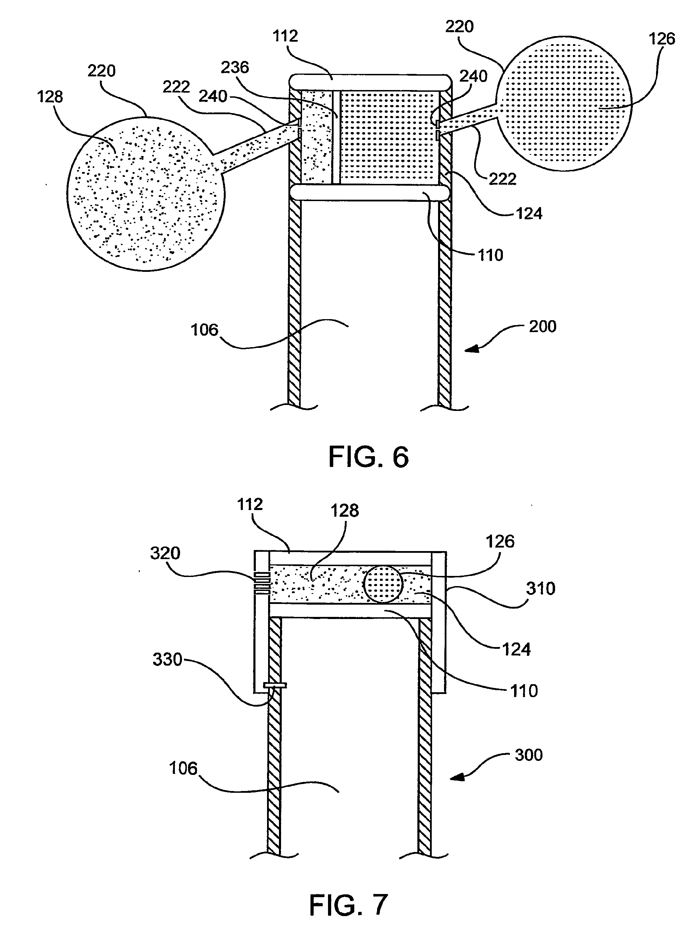

Referring to Fig. 6, a partial schematic representation of a drug delivery device employing a mechanically driven regulator is shown. In this example, expandable reservoirs or "squeeze bulbs" 220 are provided for containing each of fluids 126 and 128, respectively. Manual squeezing of one or the other of the bulbs causes the fluid from the squeezed bulb to enter space 124 and displace an equal volume of the other fluid from the space 124 and toward the reservoir (squeeze bulb) for storing that particular fluid. A piston 236 may be provided, which seals with membranes 110 and 112 to prevent fluid flow therepast, but translates with the change in volumes of the respective fluids 126 and 128. If used, the piston 236 prevents any unwanted mixing or uneven displacement of one fluid with respect to the other, particularly at the interfaces with the membranes 110, 112.

Two way pressure relief valves 240 may be provided at the interfaces between delivery tubes 222 and space 124 to prevent unwanted flow of the fluids 126, 128 into or out of space 124. The pressure relief valves may be designed to allow fluid flow therethrough only upon reaching a minimal pressure, for example about 10-45 psi. With this arrangement, the relative "cover" by the two fluids 126 and 128 will remain as intended, with no seepage or leakage of fluids into or out of the space 124. Flow will only be initiated upon squeezing one of the bulbs 220 so as to create a hydraulic pressure greater than the minimum threshold pressure for opening the pressure relief valves.

Fig. 7 is a partial schematic representation of a drug delivery device employing another mechanism for varying the relative cover of membranes 110, 112 by high resistance liquid 126. In this example, a cap 310 is provided over the end of the device 300 through which diffusion or mass transport is to occur. Cap 310 includes membrane 112 at the end thereof, with second chamber 106 having a membrane 110 mounted at the end thereof, the same as in the previously described examples. Liquid 126 in this example is permanently positioned in space 124 and has surface tension properties that cause it to tend to "bead up" when no force is applied thereto. One example of such a fluid is mercury Fluid 126 substantially prevents any osmosis/diffusion through membranes 110,112 in areas where the fluid 126 is located between the membranes. The remainder of the space 124 is filled with fluid 128, which, as described above, has substantially insignificant resistance to diffusion/osmosis.

In order to adjust the relative cover by fluid 126 in this example, cap 310 is translated in a direction along the longitudinal axis "1" of the device 300. When translated "downwardly" as shown in Fig. 7, the space 124 will reduce in volume and tend to flatten the spherical shape of the liquid 126 so as to effectively cover a greater relative area of the membranes 110,112. A liquid vent 320 is provided to allow the escape of liquid 128 as the space 124 is compressed. Liquid vent 320 may vent into the environment of use or into a separate ancillary reservoir for exchange of an inert fluid 128. However, liquid vent 320 is impermeable to liquid 126. Therefor, at its most compressed state, the space 124 is completely filled with liquid 126 which spreads itself to cover the entire areas of membranes 110,112, thereby effectively "shutting off" drug delivery by reducing osmosis/diffusion to a rate of zero. The position shown in Fig. 7 is the setting for the maximum rate of diffusion/osmosis and thus the maximum rate of drug delivery.

Movement of the cap 310 with respect to the remainder of the device 300 may be manually accomplished, such as by applying a compressive force to the device (e.g., by hand) to press the cap 310 down on the device by a desired amount. A series of detents or knurling (not shown) can be formed between the outer wall of the second chamber 106 and the inner wall of cap 310 so that the cap can be advanced by discrete and discernible amounts. Alternatively, mating screw threads 330 may be provided between the outer wall of the second chamber 106 and the inner wall of cap 310 so that rotation of the cap 310 relative to the remainder of device 300 will cause translation of the cap in one of two directions along the longitudinal axis (relative to clockwise and counterclockwise rotation, respectively). Movements of the cap 310 away from second chamber 106 need to be accomplished while liquid vent 310 is submersed in an environment of liquid 128 so that liquid 128 can fill the expanding area of space 124.

**Claims**

1. An implantable drug delivery device (100) comprising:

   a body (102) adapted to contain at least one drug therein for dispensing therefrom; **characterised by**:

   at least one membrane (110) adapted to selectively pass one or more liquids (14) therethrough, said at least one membrane (110) having a cross-sectional area adapted to be exposed to the one or more liquids for transport thereof; and
   at least one cover member (126, 128) movable with respect to said at least one membrane (110) to cover at least a portion of said at least one membrane (110), said cover member (126, 128) being movable so as to vary the cross sectional area of said at least one membrane (110) that is exposed for transport of the one or more liquids.

2. The implantable drug delivery device of claim 1, wherein said at least one cover member comprises a cover member (40) with substantially infinite resistance to transport of said one or more liquids, and wherein movement of said cover member (40) over said membrane (20) effectively reduces said cross-sectional area.

3. The implantable drug delivery device of claim 2, wherein said cover member with substantially infinite resistance comprises a first cover member (40), said device further comprising a second cover member (30) having substantially insignificant resistance to transport of said one or more liquids, relative to a resistance of said at least one membrane, said second cover member (30) located adjacent said first cover member (40) and covering any portion of said cross-sectional area not covered by said first cover member (40).

4. The implantable drug delivery device of claim 3, wherein said at least one membrane comprises a pair of membranes (110, 112) aligned in parallel and defining a gap (124) therebetween, said first (126) and second (128) covers being adapted to pass through said gap (124) to effectively cover said cross-sectional area.

5. The implantable drug delivery device of claim 4, further comprising a labyrinth defined between said pair of membranes and adapted to channel said covers therethrough.

**6.** The implantable drug delivery device of claim 4, wherein each of said pair of membranes (110, 112) have said cross-sectional area, each said cross-sectional area being substantially equal and effected simultaneously by said cover members.

**7.** The implantable drug delivery device of claim 1, wherein said at least one membrane allows inflow of fluids to drive said at least one drug from said body.

**8.** The implantable drug delivery device of claim 2, wherein said at least one cover member comprises a fluid.

**9.** The implantable drug delivery device of claim 8, wherein said at least one cover member comprises a magnetic fluid.

**10.** The implantable drug delivery device of claim 3, wherein said first cover member comprises a first fluid (126) and wherein said second cover member comprises a second fluid (128) different from said first fluid.

**11.** The implantable drug delivery device of claim 10, wherein at least one of said first and second fluids comprises a magnetic fluid (126).

**12.** The implantable drug delivery device of claim 1, wherein said at least one cover member is arranged to be driven remotely, after implantation of said device in an implantation site, from a location external of the implantation site.

**13.** The implantable drug delivery device of claim 12, wherein said at least one cover member is magnetically drivable.

**Patentansprüche**

**1.** Implantierbare Arzneimittelverabreichungsvorrichtung (100), die umfasst:

einen Körper (102), der dazu ausgelegt ist, wenigstens ein Arzneimittel zu enthalten, um es hiervon abzugeben; **gekennzeichnet durch**:

wenigstens eine Membran (110), die dazu ausgelegt ist, wahlweise eine oder mehrere Flüssigkeiten (14) durchzulassen, wobei die wenigstens eine Membran (110) eine Querschnittsfläche besitzt, die dazu ausgelegt ist, mit der einen oder den mehreren Flüssigkeiten beaufschlagt zu werden, um sie zu transportieren; und

wenigstens ein Abdeckelement (126, 128), das in Bezug auf die wenigstens eine Membran (110) beweglich ist, um wenigstens einen Abschnitt der wenigstens einen Membran (110) abzudecken, wobei das Abdeckelement (126, 128) beweglich ist, um die Querschnittsfläche der wenigstens einen Membran (110), die mit der einen oder den mehreren Flüssigkeiten beaufschlagt wird, um sie zu transportieren, zu verändern.

**2.** Implantierbare Arzneimittelverabreichungsvorrichtung nach Anspruch 1, wobei das wenigstens eine Abdeckelement ein Abdeckelement (40) mit einem im Wesentlichen unendlichen Widerstand für den Transport der einen oder der mehreren Flüssigkeiten aufweist und wobei die Bewegung des Abdeckelements (40) über die Membran (20) die Querschnittsfläche effektiv reduziert.

**3.** Implantierbare Arzneimittelverarbreichungsvorrichtung nach Anspruch 2, wobei das Abdeckelement mit dem im Wesentlichen unendlichen Widerstand ein erstes Abdeckelement (40) umfasst, wobei die Vorrichtung ferner ein zweites Abdeckelement (30) mit einem im Wesentlichen unbedeutenden Widerstand für den Transport der einen oder der mehreren Flüssigkeit relativ zu dem Widerstand der wenigstens einen Membran umfasst, wobei sich das zweite Abdekkelement (30) in der Nähe des ersten Abdeckelements (40) befindet und irgendeinen Abschnitt der Querschnittsfläche, der nicht durch das erste Abdeckelement (40) abgedeckt ist, abdeckt.

**4.** Implantierbare Arzneimittelverabreichungsvorrichtung nach Anspruch 3, wobei die wenigstens eine Membran ein Paar Membranen (110, 112) umfasst, die parallel ausgerichtet sind und zwischen sich einen Spalt (124) definieren, wobei die erste Abdeckung (126) und die zweite Abdeckung (128) dazu ausgelegt sind, sich durch den Spalt (124) zu bewegen, um die Querschnittsfläche effektiv abzudecken.

**5.** Implantierbare Arzneimittelverabreichungsvorrichtung nach Anspruch 4, die ferner ein Labyrinth umfasst, das zwischen dem Paar Membranen definiert ist und dazu ausgelegt ist, die Abdeckungen hindurch zu führen.

**6.** Implantierbare Arzneimittelverabreichungsvorrichtung nach Anspruch 4, wobei jede der beiden Membranen (110, 112) diese Querschnittsfläche besitzt, wobei jede solche Querschnittsfläche im Wesentlichen gleich ist und durch die Abdeckelemente gleichzeitig geschaffen wird.

**7.** Implantierbare Arzneimittelverabreichungsvorrichtung nach Anspruch 1, wobei die wenigstens eine Membran einen Zufluss von Fluiden zulässt, um das wenigstens eine Arzneimittel aus dem Körper zu drängen.

**8.** Implantierbare Arzneimittelverabreichungsvorrichtung nach Anspruch 2, wobei das wenigstens eine Abdeckelement ein Fluid enthält.

**9.** Implantierbare Arzneimittelverabreichungsvorrichtung nach Anspruch 8, wobei das wenigstens eine Abdeckelement ein magnetisches Fluid enthält.

**10.** Implantierbare Arzneimittelverabreichungsvorrichtung nach Anspruch 3, wobei das erste Abdeckelement ein erstes Fluid (126) enthält und wobei das zweite Abdeckelement ein zweites Fluid (128), das von dem ersten Fluid verschieden ist, enthält.

**11.** Implantierbare Arzneimittelverabreichungsvorrichtung nach Anspruch 10, wobei das erste Fluid und/oder das zweite Fluid ein magnetisches Fluid (126) umfassen.

**12.** Implantierbare Arzneimittelverabreichungsvorrichtung nach Anspruch 1, wobei das wenigstens eine Abdeckelement dazu ausgelegt ist, von einem Ort außerhalb des Implantationsorts fernangetrieben zu werden, nachdem die Vorrichtung an dem Implantationsort implantiert worden ist.

**13.** Implantierbare Arzneimittelverabreichungsvorrichtung nach Anspruch 12, wobei das wenigstens eine Abdeckelement magnetisch angetrieben werden kann.

**Revendications**

**1.** Dispositif implantable (100) pour l'administration de médicaments, comprenant :

un corps (102) adapté pour contenir au moins un médicament à administrer depuis celui-ci ; **caractérisé par** :

au moins une membrane (110) adaptée pour laisser passer de manière sélective un ou plusieurs liquide (s) (14), chaque membrane (110) ayant une aire de section transversale adaptée pour être exposée aux liquides pour le transport de ceux-ci ; et
au moins un élément protecteur (126, 128) mobile par rapport à ladite membrane (110) pour couvrir au moins une partie de ladite membrane (110), ledit élément protecteur (126, 128) étant mobile de façon à faire varier l'aire de section transversale de ladite membrane (110) qui est exposée pour le transport du ou des liquide(s).

**2.** Dispositif implantable pour l'administration de médicaments selon la revendication 1, dans lequel ledit élément protecteur comprend un élément protecteur (40) ayant une résistance substantiellement infinie au transport desdits liquides, et dans lequel le mouvement dudit élément protecteur (40) sur ladite membrane (20) réduit effectivement ladite aire de section transversale.

**3.** Dispositif implantable pour l'administration de médicaments selon la revendication 2, dans lequel ledit élément protecteur à résistance substantiellement infinie comprend un premier élément protecteur (40), ledit dispositif comprenant en outre un deuxième élément protecteur (30) ayant une résistance substantiellement insignifiante pour le transport desdits liquides, par rapport à une résistance de ladite au moins une membrane, ledit deuxième élément protecteur (30) étant adjacent audit premier élément protecteur (40) et couvre toute partie de ladite aire de section transversale non couverte par ledit premier élément protecteur (40).

**4.** Dispositif implantable pour l'administration de médicaments selon la revendication 3, dans lequel ladite membrane comprend une paire de membranes (110, 112) alignées en parallèle et définissant un espace (124) entre elles, lesdits premier (126) et deuxième (128) couvercles étant adaptés pour passer dans ledit espace (124) pour couvrir effectivement ladite aire de section transversale.

**5.** Dispositif implantable pour l'administration de médicaments selon la revendication 4, comprenant en outre un labyrinthe défini entre lesdites membranes de ladite paire et adapté pour canaliser lesdits couvercles.

**6.** Dispositif implantable pour l'administration de médicaments selon la revendication 4, dans lequel chaque membrane de ladite paire (110, 112) a ladite aire de section transversale, toutes lesdites aires de section transversale étant sensiblement égales et effectuées simultanément par lesdits éléments protecteurs.

**7.** Dispositif implantable pour l'administration de médicaments selon la revendication 1, dans lequel ladite au moins une membrane permet l'entrée de fluides pour entraîner ledit au moins un médicament hors dudit corps.

**8.** Dispositif implantable pour l'administration de médicaments selon la revendication 2, dans lequel ledit au moins un élément protecteur comprend un fluide.

**9.** Dispositif implantable pour l'administration de médicaments selon la revendication 8, dans lequel ledit au moins un élément protecteur comprend un fluide magnétique.

**10.** Dispositif implantable pour l'administration de médicaments selon la revendication 3, dans lequel ledit premier élément protecteur comprend un premier fluide (126) et dans lequel ledit deuxième élément protecteur comprend un deuxième fluide (128) différent dudit premier fluide.

**11.** Dispositif implantable pour l'administration de médicaments selon la revendication 10 dans lequel au moins l'un desdits premier et deuxième fluides comprend un fluide magnétique (126).

**12.** Dispositif implantable pour l'administration de médicaments selon la revendication 1, dans lequel ledit au moins un élément protecteur est agencé pour être entraîné à distance, après implantation dudit dispositif dans un site d'implantation, depuis un lieu extérieur au site d'implantation.

**13.** Dispositif implantable pour l'administration de médicaments selon la revendication 12 dans lequel ledit au moins un élément protecteur peut être entraîné de façon magnétique.

**FIG. 1**

$R_2 << R_1$
$R_3 >> R_1$

**FIG. 2**

FIG. 3

FIG. 4

FIG. 5

FIG. 6

FIG. 7

**EP 1 435 920 B1**

**Patent documents cited in the description**

- US 4692147 A
- WO 0126708 A
- US 5169383 A
- US 5951538 A
- US 5567287 A
- US 4886514 A
- US 5593552 A
- US 5538605 A
- US 5454922 A
- US 5707499 A
- US 5855761 A
- US 4865845 A